# FASCICULE DE BREVET EUROPEEN

(11) **EP 0 407 237 B1**
(45) Date de publication et mention de la délivrance du brevet: **29.11.1995**
(21) Numéro de dépôt: 90401506.2
(22) Date de dépôt: 05.06.1990
(51) Int. Cl.: C07D 498/20, G03C 1/685

(54) **Composés photochromiques de type indolino-spiro-oxazine, leur procédé de préparation, compositions et article photochromiques contenant de tels composés**
Fotochrome Indolino-spiro-oxazine, Verfahren zu ihrer Herstellung und diese Verbindungen enthaltende fotochrome Zusammensetzungen und Gegenstände
Indolino-spiro-oxazine photochromic compounds, process for their preparation and photochromic compositions and materials containing them

(30) Priorité: 05.06.1989 FR 8907401
(43) Date de publication de la demande: 09.01.1991
(73) Titulaire: ESSILOR INTERNATIONAL (Compagnie Générale d'Optique), F-94220 Charenton-le-Pont (FR)
(72) Inventeur: Guglielmetti, Robert, F-13009 Marseille (FR); Tardieu, Pascale, F-13004 Marseille (FR)
(74) Mandataire: Thibon-Littaye, Annick

(56) Documents cités:
- EP-A- 0 245 020
- EP-A- 0 313 941
- WO-A-87/00524

## Description

La présente invention concerne de nouveaux composés photochromiques du type indolino-spiro-oxazine, leur procédé d'obtention, ainsi que des compositions et articles à propriétés photochromiques renfermant au moins un desdits composés photochromiques. De manière générale, les composés de la présente invention peuvent être utilisés avantageusement dans la fabrication de toute lentille optique, une lentille optique désignant notamment une lentille ophtalmique, une lentille de contact ou une lentille de protection solaire.

Le photochromisme est un phénomène réversible bien connu, illustré par exemple par un composé qui change de couleur quand il est exposé à des radiations lumineuses dont certaines appartiennent au domaine de l'U.V., notamment les radiations solaires, et qui reprend sa couleur initiale lorsqu'on interrompt l'exposition lumineuse.

De tels composés sont utilisés par exemple dans la fabrication de lentilles pour lunettes de protection solaire ou dans d'autres applications où intervient le besoin de faire varier la transparence d'un article en fonction de l'intensité lumineuse environnante. Ils peuvent être appliqués sur un support transparent ou incorporés dans un matériau organique polymérisé transparent en combinaison avec une très grande variété de compositions polymériques.

Un certain nombre de composés photochromiques organiques comportant dans leur formule un groupe indolino-spiro-oxazine ont déjà été proposés. Ils ont rencontré des succès divers dans la pratique.

Ainsi, les brevets américains US 3 562 172 et 3 578 602 ont divulgué l'effet photochromique de certains composés appartenant à la famille des indolino-spiro-naphtoxazines, tandis que le brevet américain US 4 215 010 décrit des indolino-spironaphtoxazines dont le cycle naphtalène est substitué par des groupements méthoxy, éthoxy ou un atome d'halogène.

Des composés photochromiques analogues comportant un cycle pyridobenzène au lieu du cycle naphtalène des composés ci-dessus sont décrits dans le brevet américain US 4 720 547, ainsi que dans la demande internationale WO 87/00524. Cette dernière donne pour ces composés une formule générale impliquant dans la partie oxazine un groupe hétéro-aromatique comportant 1 ou 2 atomes d'azote inclus dans 2 ou 3 cycles, conformément à une formule générale du type suivant : 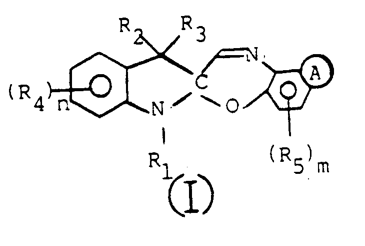
dans laquelle le cycle A est un hétérocycle aromatique à six chaînons, comportant un ou deux atomes d'azote.

Toutefois les seuls composés particuliers qui aient été préparés et essayés dans cette demande de brevet comportent conme groupe hétéro-aromatique un groupe pyridobenzène, benzopyridobenzène ou dipyridobenzène.

Les différents cycles des parties indoline et oxazine des composés photochromiques connus peuvent porter des substituants divers, avec en particulier, n variant de 0 à 4 et m prenant les valeurs 1 ou 2 :
- R₁ peut représenter :
   i) un groupe alkyle de 1 à 16 atomes de carbone tel qu'un groupe méthyle, éthyle, n-propyle, isopropyle, n-butyle ;
   ii) un groupe allyle, phényle, arylalkyle tel qu'un groupe benzyle, phényle mono et di-substitué par des substituants du type alkyle ou alkoxy de 1 à 6 atomes de carbone ;
   iii) un groupe alicyclique tel qu'un groupe cyclohexyle, éventuellement substitué ;
   iv) un groupe hydrocarbure aliphatique comportant dans sa chaîne un ou plusieurs hétéroatomes tels que O, N ou S, notamment une fonction acide, ester ou alcool ;
- R₂ et R₃ peuvent chacun et indépendamment l'un de l'autre représenter un atome d'hydrogène, un groupe C₁₋₈ alkyle, phényle, phényle mono et disubstitué par des groupes C₁₋₄ alkyle et/ou C₁₋₅ alkoxy ou peuvent être combinés pour former une chaîne cyclique de 6 à 8 atomes de carbone (incluant le carbone spirannique 3 de l'hétérocycle indolinique) ;
- R₄ et R₅ peuvent chacun et indépendamment l'un de l'autre représenter :
   i) un atome d'hydrogène, une fonction amine NR'R'', où R' et R'' représentent chacun et indépendamment un atome d'hydrogène, un groupe alkyle, cycloalkyle, phényle ou un dérivé substitué de celui-ci ; R' et R'' peuvent se combiner pour former un cycloalkyle pouvant être substitué et contenir un ou plusieurs hétéroatomes ;
   ii) un groupe R, OR, SR, COR ou COOR dans lequel R représente un atome d'hydrogène, un groupe alkyle de 1 à 6 atomes de carbone ou un groupe aryle ou hétéroaryle ;
   iii) un atome d'halogène, un groupe C₁₋₄ monohaloalkyle, un groupe C₁₋₄ polyhaloalkyle tel que CF₃, l'halogène étant notamment Cl, Br ;
   iv) -NO₂, CN, SCN ;
chacun des substituants R₄ pouvant être présent sur l'un quelconque des atomes de carbone convenables de la partie indoline du composé photochromique.

De préférence, toutefois dans cette formule I :
- R₁ est un groupe C₁₋₄ alkyle, phényle ou benzyle ;
- R₂ et R₃ sont choisis parmi les groupes C₁₋₅ alkyle tels que méthyle et éthyle ou un groupe phényle, ou sont combinés pour former un groupe cyclohexyle ;
- chacun des groupes R₄ est choisi parmi le groupe constitué par hydrogène, C₁₋₂ alkyle, chlore, fluor, brome, iode, C₁₋₂ trihaloalkyle et C₁₋₅ alkoxy ;
- et R₅ est un atome d'hydrogène ou un groupe C₁₋₄ alkoxy ou une ami ne tertiaire ou un halogène.

L'invention apporte des perfectionnements aux composés photochromiques de formule (I), par une amélioration des propriétés recherchées dans leurs applications les plus courantes, notamment pour ce qui concerne la compatibilité avec les matériaux organiques de lentilles transparentes de protection solaire et la persistance de la coloration recherchée en cas d'exposition de longue durée à la lumière, en particulier dans le cadre de leur utilisation pour rendre photochromique des vernis de type polysiloxane.

L'invention propose à cet effet d'utiliser en partie oxazine de la molécule un hétérocycle insaturé à six chaînons de type pyrimidine, dans un noyau de type quinazoline.

Ainsi l'invention a pour objet des composés photochromiques caractérisés en ce qu'ils présentent la formule développée suivante : 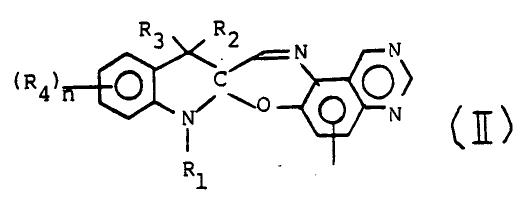
dans laquelle :
- n varie de 0 à 4 ;
- R₁ représente un groupe alkyle tel qu'un groupe méthyle, éthyle, isopropyle, n-butyle ;
- R₂ et R₃ représentent chacun et indépendamment l'un de l'autre un groupe méthyle ou éthyle ou phényle ;
- R₄ est un atome d'hydrogène, un groupe méthyle, méthoxy ou chloro ;
Parmi les composés selon l'invention sont particulièrement intéressants les composés dans lesquels :
- la partie oxazine étant de type pyrimido-benzène et R₅ étant H,
- R₁ est un groupe méthyle ou isopropyle ;
- R₂ et R₃ représentent chacun un groupe méthyle ;
- R₄ est un atome d'hydrogène, un groupe méthyle, méthoxy ou chloro.

La présente invention a également pour objet un procédé de préparation des composés indolinospiro-oxazine photochromiques représentés par la formule (II).

Il exploite pour une part un schéma général de synthèse des hétérocycles de type (I) qui est connu et qui consiste à réaliser la condensation d'une base de Fischer ou composé alkylidène-2 indolinique sur un composé hydroxynitroso-hétéro-aromatique. Cependant les composés intermédiaires hétéro-aromatiques nécessaires pour aboutir aux composés selon l'invention sont en eux-mêmes nouveaux et ils n'ont jamais été synthétisés à ce jour.

Conformément à l'invention, le composé quinazoline convenable peut être préparé à partir d'un composé hydroxy-3 benzaldéhyde, substitué éventuellement par un ou plusieurs radicaux R₅ tels que définis pour la formule II, par un procédé qui comporte la nitration de ce composé, la réaction de l'hydroxy-3 nitro-6 benzaldéhyde obtenu avec de la formamide pour obtenir le dérivé benzylidène-bisformamide correspondant, la transformation de celui-ci en hydroxy-6 quinazoline, et la nitrosation de ce dernier composé.

Dans leur mise en oeuvre pour la préparation de compositions photochromiques, les composés selon la présente invention peuvent être dissous dans un solvant convenable tel que le toluène ou l'éthanol afin d'obtenir une solution photochromique. Ces mêmes composés photochromiques peuvent également être dissous au sein d'un polymère, d'un copolymère ou d'un mélange de polymères en solution dans un solvant organique convenable.

Ils entrent ainsi dans la constitution de compositions selon la présente invention, qui peuvent être appliquées ou introduites dans ou sur un matériau polymère organique transparent pour obtenir un article transparent photochromique. Préférentiellement, ce matériau est un matériau de qualité optique, plus particulièrement un matériau convenable pour la fabrication de lentilles ophtalmiques.

Ils peuvent aussi entrer dans des compositions photochromiques faisant également l'objet de la présente invention qui peuvent être utilisées directement dans la constitution de films plastiques photochromiques, de plaques et de lentilles telles que des lentilles pour lunettes de soleil, viseurs, optiques de caméra et filtres.

Des exemples de compositions appropriées, conformément à l'invention, pour la fabrication de matériaux et articles transparents photochromiques, comportent un ou plusieurs des composés photochromiques de la présente invention en combinaison avec un ou plusieurs des polymères suivants :
polymère d'un monomère de polyolallylcarbonate, poly-acrylate, polyalkyl-acrylates tels que le polyméthacrylate de méthyle (PMMA), acétate de cellulose, triacétate de cellulose, propiono et butyro-acétate de cellulose, acétate de polyvinyle, alcool polyvinylique, polyuréthanes, polycarbonates, polyéthylènetéréphtalate, polystyrène, copolymères de styrène et de méthacrylate de méthyle, acrylonitrile, polyvinylbutyral.

La quantité de composé photochromique (ou de composition contenant ce composé) appliquée ou introduite sur ou dans le matériau polymère n'est pas d'importance critique et elle dépend généralement de l'intensité de couleur désirée sous irradiation et de la méthode utilisée pour incorporer ou appliquer le composé photochromique. Cette dernière peut être choisie parmi les nombreuses méthodes applicables aux composés photochromiques de l'art antérieur, parmi lesquelles notamment la dissolution ou la dispersion du composé dans la composition de base du matériau, ou la réalisation d'une couche photochromique en surface ou à l'intérieur d'un matériau support transparent.

D'une manière générale, plus on ajoute de compose photochromique, plus la coloration sous irradiation sera importante. Une telle quantité peut être décrite comme une quantité photochromique. De façon usuelle, la quantité de composé photochromique incorporée au matériau optique est de 0,01 à 20 % en poids, et préférentiellement de 0,05 à 10 % en poids, par rapport au poids total de matériau optique.

On obtient ainsi des effets photochromiques qui se traduisent par l'apparition d'une coloration sous exposition à des radiations appartenant au domaine de l'U.V., avec retour à la couleur ou la transparence originelle lorsqu'on interrompt l'exposition aux radiations U.V. Ce changement de coloration peut se renouveler un très grand nombre de fois, comme il est demandé pour des lunettes de protection solaire. De plus, la coloration persiste pendant tout le temps de l'exposition au rayonnement solaire mieux que dans le cas des composés photochromiques de l'art antérieur.

L'invention sera maintenant plus complètement illustrée au moyen d'exemples particuliers de mise en oeuvre non limitatifs.

### EXEMPLE 1

### - Phase I

Etant rappelé que l'on sait préparer des indolino-spiro-oxazines de formule I, par exemple à partir d'un composé méthylène-2-indolinique et d'un composé nitroso-5 hydroxy-6 hétéroaromatique, on décrit dans cette première phase la préparation d'un composé intermédiaire de type nitroso-5 hydroxy-6 quinazoline, conformément au schéma réctionnel A ci-après, où R₅ a la même signification que dans la formule II des composés photochromiques de l'invention : 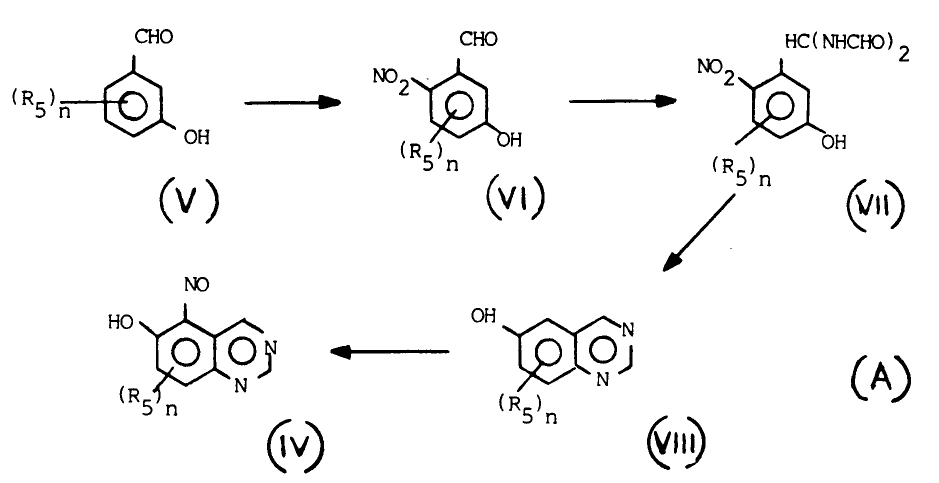
Le composé de départ présente la formule V, où R₅ est H dans l'exemple particulier.

On ajoute lentement 3 g d'hydroxy-3 benzaldéhyde (formule V) à 30 ml d'acide nitrique (d = 1,17, solution à 28 %). La température de la solution est maintenue entre 35 °C et 45 °C. A la fin de cet ajout, l'hydrolyse est terminée et le mélange réactionnel est alors laissé au repos à la température ordinaire. Un précipité jaune est ainsi obtenu ; il est filtré puis traité à reflux dans du benzène (20 ml) pendant 15 à 20 mn. La partie insoluble est extraite et recristallisée dans l'eau. On obtient l'hydroxy-3 nitro-6 benzaldéhyde (formule VI) avec un rendement final de 25 %.
F = 167 °C (C₇H₅NO₄, M = 167).

Dans l'étape suivante, on fait barboter HCl gazeux activement dans un mélange de 4 g d'hydroxy-3 nitro-6 benzaldéhyde et 30 ml de formamide. Quand la température atteint 100 °C, on arrête le barbotage gazeux et on laisse la solution au repos à la température ordinaire pendant une nuit. La matière solide est lavée à l'éther puis introduite dans 10 ml d'eau glacée et le pH est ajusté à 3 par NaOH 6N. Le produit brut est purifié par recristallisation dans l'eau. Rendement 84 %, F = 244 °C (C₉H₉N₃O₅, M = 239).

A un mélange de 2 g d'hydroxy-3 nitro-6 benzylidène bis-formamide ainsi obtenu (formule VII) avec de la poussière de zinc (6 g) et de la glace pilée (24 g), on ajoute sous agitation 8 ml d'acide acétique en 5 mn. On poursuit l'agitation de la solution à température ordinaire pendant 30 mn (la température s'élève à 41 °C) puis pendant 2 h en rajoutant du zinc (3 g) pendant la première heure. La solution est filtrée puis extraite doucement par de l'éther oxyde (pH = 7-8). Après évaporation de l'éther le pH est ajusté à 5 au moyen d'une solution NaOH 3N. Le produit solide marron récupéré (hydroxy-6 quinazoline, formule VIII) est recristallisé dans l'eau. Rendement 57 %,
F = 239 °C (C₈H₆N₂, M = 146).

Sous agitation et en 1 h, on ajoute une solution de 0,14 mole de nitrite de sodium dans 30 ml d'eau, à une solution froide (0-5 °C) de 0,14 mole d'hydroxy-6 quinazoline, 12 ml d'acide chlorhydrique concentré et 50 ml d'eau distillée. On poursuit l'agitation pendant 1 h. On obtient ainsi la nitroso-5 hydroxy-6 quinazoline (formule IV) sous la forme d'un précipité jaune qui est lavé à l'eau puis séché.
Rendement 60 %, F = 172 °C (C₈H₅N₃O₂, M = 175).

### - Phase II

Le composé intermédiaire de formule IV obtenu lors de la première phase est utilisé pour préparer un composé photochromique de formule II où R₄ est H et R₁, R₂, R₃ sont tous trois des groupes méthyle, comme suit :
1,4 10⁻³ moles d'une base de Fischer triméthyl-1,3,3 méthylène-2 indoline dissoutes dans 10 ml de n-heptane et 2 ml d'éthanol sec sont chauffées à reflux. A température constante (environ 76 °C), on ajoute lentement, en 2 h, 1,4 10⁻³ moles de nitroso-5 hydroxy-6 quinazoline en suspension dans 12 ml d'éthanol sec.

Le mélange réactionnel est maintenu à reflux pendant 45 mn. Tandis que la réaction progresse lentement, on élimine l'eau avec l'éthanol par un dispositif de Dean Stark et on ajoute ensuite 30 ml d'éthanol pour atteindre un volume constant de solvant.

Après avoir fait passer le mélange à travers une colonne chromatographique de gel de silice et éliminé le solvant, on recristallise le résidu solide dans un mélange approprié de solvants apolaires tels que l'éther de pétrole, l'éther-oxyde, le n-hexane ou le benzène.

Le produit obtenu est constitué par l'indolino-spiro-oxazine de formule II, soit la triméthylindolino-spiro-quinazolino-oxazine. Le rendement est de l'ordre de 50 à 60 % quand le solvant réactionnel est un mélange de n-heptane et éthanol. Une purification ultérieure par recristallisation ramène à 40 % le rendement final.
Point de fusion F = 169 °C ( C₂₀H₁₈N₄O M = 330 ).

### EXEMPLE II

En suivant les mêms voies de synthèse que dans l'Exemple I, on obtient le composé de formule II où R₁ est iC₃H₇, R₂ = R₃ = méthyle, R₄ est H, R₅ est H, en partant de la nitroso-5 hydroxy-6 quinazoline et de la base de Fischer isopropyl-1 méthylène-2 diméthyl-3,3 indoline.
F = 180 °C ( C₂₂H₂₂N₄O M = 358 ).

### EXEMPLE III

De même, on obtient le composé de formule II où R₁ est CH₃, de même que R₂ et R₃, R₄ est un groupe méthoxy en position 5 du cycle indoline et R₅ est H, à partir de la nitroso-5 hydroxy-6 quinazoline et de la base de Fischer triméthyl-1,3,3 méthylène-2 méthoxy-5 indoline.
Composé final : C₂₁ H₂₀ N₄ O₂ F = 140 °C

### EXEMPLE IV

La triméthylindolino-spiro-quinazolino-oxazine obtenue dans l'Exemple I est introduite dans un vernis polysiloxane du type de ceux décrits dans le brevet français FR 82 0440, à raison de 1 % en poids. Le vernis obtenu est appliqué sur des lentilles ophtalmiques en matériau organique, puis durci pendant 2 h à 100 °C. L'épaisseur des couches obtenues est de 2 microns. Le pourcentage en poids de composé photochromique dans la couche durcie est de l'ordre de 5 %.

On soumet les lentilles ainsi revêtues à des essais d'irradiation au moyen d'un appareil connu commercialement sous le nom de SUNTEST ORIGINAL HANAU dans les conditions suivantes :
- Emetteur : source au xénon ;
- Rayonnement énergétique de 1000 W/m² dans un domaine de longueurs d'ondes comprises entre 300 et 830 nm ;
- Eclairement : 150 kilolux ;
- Température environnante : 40 °C.

L'appréciation du photochromisme sous irradiation s'effectue visuellement toutes les deux heures. On constate que l'effet photochromique persiste après 4 heures d'irradiation.

A titre d'exemples comparatifs, on a soumis aux mêmes essais des vernis préparés de la même manière avec des composés photochromiques de l'art antérieur, et l'on a pu observer que :
1) pour la triméthyl-indolino-spiro-naphto-oxazine du brevet américain US 3 578 602, l'effet photochromique a disparu dès 2 heures d'irradiation ;
2) pour la triméthyl-indolino-spiro-quinolino-oxazine du brevet américain US 4 720 547, l'effet photochromique faiblit à 2 heures d'irradiation et s'éteint complètement avant 4 heures d'irradiation.

## Revendications (Revendications pour l'(les) Etat(s) contractant(s) suivant(s): CH, DE, GB, IT, LI)

1. Composés photochromiques, caractérisés en ce qu'ils présentent la formule développée suivante : 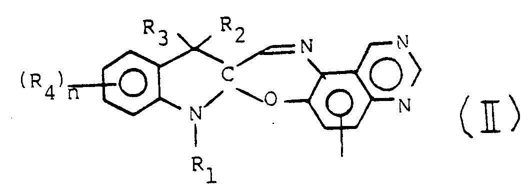 dans laquelle :
- n varie de 0 à 4 ;
- R1 représente un groupe alkyle tel qu'un groupe méthyle, éthyle, isopropyle, n-butyle ;
- R₂ et R₃ représentent chacun et indépendamment l'un de l'autre un groupe méthyle ou éthyle ou phényle ;
- R₄ est un atome d'hydrogène, un groupe méthyle, méthoxy ou chloro ;

2. Composés photochromiques suivant la revendication 1, caractérisés en ce qu'ils sont constitués par un ou plusieurs composés de type trialkylindolino-spiro-quinazolino-oxazine, présentant la formule II dans laquelle :
- R₁ est un groupe méthyle ou isopropyle ;
- R₂ et R₃ sont des groupes méthyle ;
- R₄ est H ou -OCH₃.

3. Compositions photochromiques pour lentilles optiques contenant au moins un des composés suivant l'une quelconque des revendications 1 à 2, en proportion photochromique.

4. Vernis photochromique de type polysiloxane, caractérisé en ce qu'il comporte une quantité photochromique d'un ou plusieurs composés suivant l'une quelconque des revendications 1 à 2.

5. Vernis photochromique de type polysiloxane, caractérisé en ce qu'il comporte une quantité photochromique d'un ou plusieurs composés de type trialkylindolino-spiro-quinazolino-oxazine, présentant la formule II de la revendication 1 dans laquelle :
- R₁ est un groupe méthyle ou isopropyle ;
- R₂ et R₃ sont des groupes méthyle ;
- R₄ est H ou OCH₃.

6. Procédé de préparation des composés selon l'une quelconque des revendications 1 à 2, caractérisé en ce qu'il comporte la nitration d'un composé hydroxy-3 benzaldéhyde, la réaction de l'hydroxy-3 nitro-6 bensaldéhyde obtenu avec de la formamide pour obtenir le dérivé benzylidène-bis-formamide correspondant, la transformation de celui-ci en hydroxy-6 quinazoline et la nitrosation de ce dernier composé, et en ce qu'il comporte ensuite, de manière en soi connue, la condensation sur le composé hydroxy-nitroso hétéro-aromatique obtenu d'une base de Fischer ou composé alkylidène-2 indolinique.

## Revendications (Revendications pour l'(les) Etat(s) contractant(s) suivant(s): ES)

1. Procédé de préparation de composés photochromiques, caractérisé en ce qu'il comporte la nitration d'un composé hydroxy-3 benzaldéhyde, la réaction de l'hydroxy-3 nitro-6 benzaldéhyde obtenu avec de la formamide pour obtenir le dérivé benzylidène-bis-formamide correspondant, la transformation de celui-ci en hydroxy-6 quinazoline et la nitrosation de ce dernier composé, et ensuite, de manière en soi connue, la condensation sur le composé hydroxy-nitroso hétéro-aromatique obtenu d'une base de Fischer ou composé alkylidène-2 indolinique, de manière à obtenir un composé répondant à la formule développée suivante : 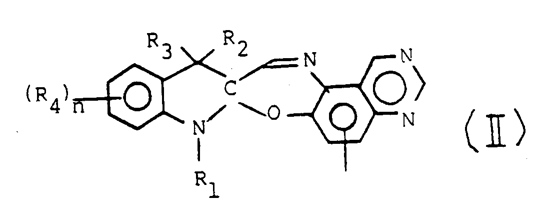 dans laquelle :
- n varie de 0 à 4 ;
- R1 représente un groupe alkyle tel qu'un groupe méthyle, éthyle, isopropyle, n-butyle ;
- R₂ et R₃ représentent chacun et indépendamment l'un de l'autre un groupe méthyle ou éthyle ou phényle ;
- R₄ est un atome d'hydrogène, un groupe méthyle, méthoxy ou chloro ;

2. Procédé suivant la revendication 1, caractérisé en ce qu'il conduit par un ou plusieurs composés de type trialkylindolino-spiro-quinazolino-oxazine, présentant la formule II de la revendication 1 dans laquelle :
- R₁ est un groupe méthyle ou isopropyle ;
- R₂ et R₃ sont des groupes méthyle ;
- R₄ est H ou -OCH₃.

3. Procédé de préparation d'une composition photochromique pour lentilles optiques, caractérisé en ce que ladite composition contient au moins un composé répondant à la formule suivant l'une quelconque des revendications 1 à 2, en proportion photochromique.

4. Procédé suivant la revendication 3, caractérisé en ce que ladite composition est un vernis photochromique de type polysiloxane.

5. Procédé suivant la revendication 4, caractérisé en ce que ladite composition est un vernis photochromique de type polysiloxane, comportant une quantité photochromique d'un ou plusieurs composés de type trialkylindolino-spiro-quinazolino-oxazine, présentant la formule II de la revendication 1, dans laquelle :
- R₁ est un groupe méthyle ou isopropyle ;
- R₂ et R₃ sont des groupes méthyle ;
- R₄ est H ou OCH₃.

## Claims (Claims for the following Contracting State(s): CH, DE, GB, IT, LI)

1. Photochromic compounds characterized in that they show the following developped formula : 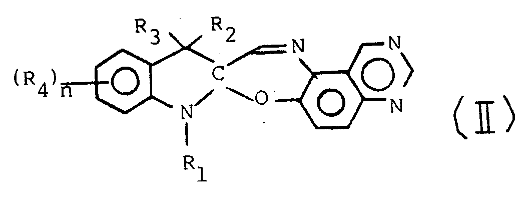 wherein
- n varies from 0 to 4 ;
- R₁ represents an alkyl group such as a methyl, ethyl, isopropyl, n-butyl group ;
- R₂ and R₃ may each independantly represent a methyl or ethyl or phenyl group ;
- R₄ is a hydrogen atom, a methyl, methoxy or chloro group.

2. Photochromic compounds according to claim 1, characterized in that they are formed of one or several compounds of the trialkylindolino-spiro-quinazolino-oxazine showing formula (II) wherein :
- R₁ is a methyl or isopropyl group ;
- R₂ and R₃ are methyl groups ;
- R₄ is H or -OCH₃.

3. Photochromic compositions for optical lenses comprising at least one compound according to claims 1 to 2 in photochromic amount.

4. A photochromic varnisxh of the polysiloxane type, characterized in that it comprises a photochromic amount of one or several compounds according to claims 1 or 2.

5. A photochromic varnish of the polysiloxane type, characterized in that it comprises a photochromic amount of one or several compounds of the trialkylindolino-spiro-quinazolino-oxazine type, showing formula (II) according to claim 1 wherein :
- R₁ is a methyl or isopropyl group ;
- R₂ and R₃ are methyl groups ;
- R₄ is H or -OCH₃.

6. A process for preparing compounds according to claims 1 or 2, characterized in that it comprises nitrating a 3-hydroxy benzaldehyde compound, reacting the 3-hydroxy 6-nitro benzaldehyde obtained with formamide to produce the corresponding benzyliden-bisformamide compound, transforming the latter into 6-hydroxy quinazoline, and nitrosating the last compound, and thereafter it comprises condensing in known manner the heteroaromatic hydroxynitroso compound obtained with a Fischer base or 2-alkyliden indolinic compound.

## Claims (Claims for the following Contracting State(s): ES)

1. A process for preparating photochromic compounds, characterized in that it comprises nitrating a 3-hydroxy benzaldehyde compound, reacting the 3-hydroxy 6-nitro benzaldehyde obtained with formamide to produce the corresponding benzyliden-bisformamide compound, transforming the latter into 6-hydroxy quinazoline, and nitrosating the last compound, and thereafter it comprises condensing in known manner the heteroaromatic hydroxynitroso compound obtained with a Fischer base or 2-alkyliden indolinic compound in manner as to obtain a compound complying with the following developed formula : 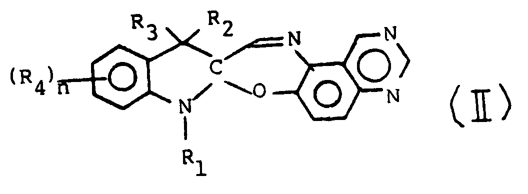 wherein :
- n varies from 0 to 4,
- R₁ represents an alkyl group such as a methyl, ethyl, isopropyl, n-butyl group ;
- R₂ and R₃ may each independantly represent a methyl or ethyl or phenyl group ;
- R₄ is a hydrogen atom, a methyl, methoxy or chloro group.

2. A process according to claim 1, characterized in that it leads to one or a several compounds of the trialkylindolino-spiro-quinazolino--oxazine type, showing formula (II) according to claim 1 wherein :
- R₁ is a methyl or isopropyl group ;
- R₂ and R₃ are methyl groups ;
- R₄ is H or -OCH₃.

3. A process for preparating a photochromic composition for lenses, characterized in that the said composition contains at least a compound complying with the formula according to the claims 1 or 2, in photochromic amount.

4. A process according to claim 3, characterized in that said composition is a photochromic varnish of polysiloxane type.

5. A process according to claim 4, characterized in that said composition is a photochromic varnish of polysiloxane type, comprising a photochromic a mount of one or several compounds of trialkylindo-spiro-quinazolino-oxazine type, showing formula (II) according to claim 1, wherein :
- R₁ is a methyl or isopropyl group ;
- R₂ and R₃ are methyl groups ;
- R₄ is H or -OCH₃.

## Patentansprüche (Patentansprüche für folgende(n) Vertragsstaat(en): CH, DE, GB, IT, LI)

1. Photochrome Verbindungen, **dadurch gekennzeichnet**, daß sie der nachfolgend angegebenen Formel entsprechen: 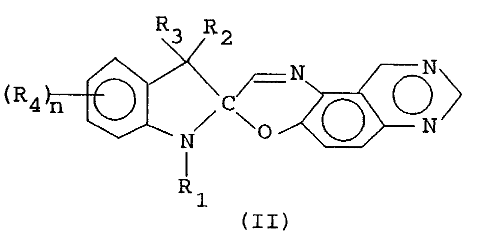 in der
n von 0 bis 4 variiert,
R₁ eine Alkylgruppe wie eine Methyl-, Ethyl-, Isopropyl-, n-Butylgruppe wiedergibt,
R₂ und R₃ jeweils und unabhängig voneinander eine Methyl-, Ethyl- oder Phenylgruppe wiedergeben,
R₄ ein Wasserstoffatom, eine Methylgruppe, Methoxygruppe oder Chlor ist.

2. Photochrome Verbindungen nach Anspruch 1, **dadurch gekennzeichnet**, daß sie aus einer oder mehreren Verbindungen vom Trialkylindolino-spiro-chinazolino-oxazintyp zusammengesetzt sind, die in Formel II wiedergegeben sind, in welcher
R₁ eine Methyl- oder Isopropylgruppe ist,
R₂ und R₃ Methylgruppen sind,
R₄ H oder -OCH₃ ist.

3. Photochrome Zusammensetzungen für optische Linsen, die mindestens eine der Verbindungen gemäß einem der Ansprüche 1 bis 2 in einem photochromen Anteil enthalten.

4. Photochromer Lack vom Polysiloxantyp, **dadurch gekennzeichnet**, daß er eine photochrome Menge von einer oder mehreren Verbindungen gemäß einem der Ansprüche 1 bis 2 umfaßt.

5. Photochromer Lack vom Polysiloxantyp, **dadurch gekennzeichnet**, daß er eine photochrome Menge von einer oder mehreren Verbindungen vom Trialkylindolino-spiro-chinazolino-oxazolintyp umfaßt, welche in Formel II des Anspruches 1 wiedergegeben sind, in welcher:
R₁ eine Methyl- oder Isopropylgruppe ist,
R₂ und R₃ Methylgruppen sind,
R₄ H oder -OCH₃ ist.

6. Verfahren zur Herstellung von Verbindungen gemäß einem der Ansprüche 1 bis 2, **dadurch gekennzeichnet**, daß eine Verbindung 3-Hydroxybenzaldehyd nitriert wird, der erhaltene 3-Hydroxy-6-nitro-benzaldehyd mit Formamid umgesetzt wird, um das entsprechende Benzyliden-bis-formamid-derivat zu erhalten, diese Verbindung in 6-Hydroxy-chinazolin umgewandelt wird und die letztere Verbindung nitrosiert wird, und anschließend in bekannter Weise die erhaltene hydroxy-nitroso-heteroaromatische Verbindung mit einer Fischerbase oder einer 2-Alkyliden-indolinverbindung kondensiert wird.

## Patentansprüche (Patentansprüche für folgende(n) Vertragsstaat(en): ES)

1. Verfahren zur Herstellung von photochromen Verbindungen, dadurch gekennzeichnet, dass eine Verbindung 3-Hydroxybenzaldehyd nitriert wird, der erhaltene 3-Hydroxy-6-nitro-benzaldehyd mit Formamid umgesetzt wird, um das entsprechende Benzyliden-bis-formamid-derivat zu erhalten, diese Verbindung in 6-Hydroxy-chinazolin umgewandelt wird und die letztere Verbindung nitrosiert wird, und anschliessend in bekannter Weise die erhaltene hydroxy-nitrosoheteroaromatische Verbindung mit einer Fischerbase oder einer 2-Alkyliden-indolinverbindung so kondensiert wird, dass eine Verbindung erhalten wird, die der nachfolgend angegebenen Formel entspricht : 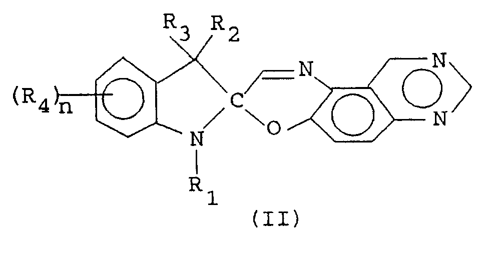 in der
n von 0 bis 4 variiert,
R₁ eine Alkylgruppe wie eine Methyl-, Ethyl-, Isopropyl-, n-Butylgruppe wiedergibt,0
R₂ und R₃ jeweils und unabhängig voneinander eine Methyl-, Ethyl- oder Phenylgruppe wiedergeben,
R₄ ein Wasserstoffatom, eine Methylgruppe, Methoxygruppe oder Chlor ist.

2. Verfahren gemäss Anspruch 1, dadurch gekennzeichnet, dass es eine oder mehrere Verbindungen vom Trialkylindolino-spiro-chinazolino-oxozintyp hervorbringt, die in Formel II wiedergegeben sind, in welcher
R₁ eine Methyl- oder Isopropylgruppe ist,
R₂ und R₃ Methylgruppen sind,
R₄ H oder -OCH₃ ist.

3. Verfahren zur Herstellung einer photochromen Zusammensetzung für optische Linsen, dadurch gekennzeichnet, dass diese Zusammensetzung mindestens eine der Verbindungen, die in Formel II gemäss einem der Ansprüche 1 bis 2 wiedergegeben sind, in einem photochromen Anteil enthält.

4. Verfahren gemäss Anspruch 3, dadurch gekennzeichnet, dass die Zusammensetzung ein photochromer Lack vom Polysiloxantyp ist.

5. Verfahren gemäss Anspruch 4, dadurch gekennzeichnet, dass die Zusammensetzung ein photochromer Lack vom Polysiloxantyp ist, der eine photochrome Menge von einer oder mehreren Verbindungen vom Trialkylindolino-spiro-chinazolino-oxazolintyp umfasst, welche in Formel II des Anspruchs 1 wiedergegeben sind, in welcher :
- R₁ eine Methyl- oder Isopropylgruppe ist,
- R₂ und R₃ Methylgruppen sind,
- R₄ H oder -OCH₃ ist.
